# EUROPÄISCHE PATENTSCHRIFT

(11) **EP 3 911 381 B1**
(45) Veröffentlichungstag und Bekanntmachung des Hinweises auf die Patenterteilung: **05.03.2025**
(21) Anmeldenummer: 20703387.9
(22) Anmeldetag: 07.01.2020
(51) Int. Cl.: A61M 5/168, A61M 5/152, A61M 5/14

(54) **MEDIZINISCHE PUMPVORRICHTUNG ZUR FÖRDERUNG EINES MEDIZINISCHEN FLUIDS**
MEDICAL PUMP DEVICE FOR CONVEYING A MEDICAL FLUID
DISPOSITIF DE POMPAGE MÉDICAL POUR TRANSPORTER UN FLUIDE MÉDICAL

(30) Priorität: 15.01.2019 DE 102019200399
(43) Veröffentlichungstag der Anmeldung: 24.11.2021
(73) Patentinhaber: B. Braun Melsungen AG, 34212 Melsungen (DE)
(72) Erfinder: HASLBECK, Karsten, 34212 Melsungen (DE)
(74) Vertreter: Patentanwälte Ruff, Wilhelm, Beier, Dauster & Partner mbB
(86) Internationale Anmeldenummer: PCT/EP2020/050218
(87) Internationale Veröffentlichungsnummer: WO 2020/148115

(56) Entgegenhaltungen:
- EP-A1- 1 486 221
- EP-A1- 3 381 489
- US-A- 4 176 683

## Beschreibung

Die Erfindung betrifft eine medizinische Pumpvorrichtung zur Förderung eines medizinischen Fluids, aufweisend eine elastomere Membran, die ein Pumpvolumen zur Aufnahme und Förderung des medizinischen Fluids ausbildet, wobei die elastomere Membran in einem wenigstens teilweise mit dem medizinischen Fluid befüllten Füllzustand des Pumpvolumens elastisch gedehnt ist, und wobei mittels der elastisch gedehnten Membran ein Förderdruck auf das Pumpvolumen zur Förderung des medizinischen Fluids in ein fluidleitend mit dem Pumpvolumen verbindbares medizinisches Fluidleitungssystem bewirkt ist, und aufweisend eine Drosseleinrichtung mit wenigstens einem Kanal, wobei der Kanal einen Einlass, der fluidleitend mit dem Pumpvolumen verbunden ist, und einen Auslass, der fluidleitend mit dem medizinischen Fluidleitungssystem verbindbar ist, aufweist, und wobei der wenigstens eine Kanal derart gestaltet ist, dass die Förderung des medizinischen Fluids durch den Auslass fertigungsseitig auf eine bestimmte Flussrate feinjustierbar ist.

Eine derartige medizinische Pumpvorrichtung ist im Bereich der Medizintechnik allgemein bekannt und zur Verwendung bei einer Infusionstherapie vorgesehen. Eine derartige Pumpvorrichtung kann auch als elastomere Infusionspumpe oder medizinische Elastomerpumpe bezeichnet werden. Die bekannte Pumpvorrichtung weist eine elastomere Membran auf, die ein Pumpvolumen zur Aufnahme und Förderung eines medizinischen Fluids ausbildet. Bei einer Befüllung des Pumpvolumens mit dem medizinischen Fluid wird die Membran ballonartig elastisch gedehnt. Die auf diese Weise gedehnte Membran bewirkt einen Förderdruck auf das Pumpvolumen. Mittels des Förderdrucks kann das medizinische Fluid in ein fluidleitend mit der Pumpvorrichtung verbindbares medizinisches Fluidleitungssystem in Form einer Katheterleitung gefördert und einem Patienten verabreicht werden. Insbesondere um eine Überdosierung des medizinischen Fluids zu vermeiden, ist es hierbei notwendig, dass das medizinische Fluid mit einer spezifischen Flussrate gefördert wird. Um dies zu gewährleisten, sieht die bekannte Pumpvorrichtung eine Drosseleinrichtung vor, die zur Drosselung der Förderung auf eine bestimmte Flussrate vorgesehen ist. Hierzu weist die Drosseleinrichtung einen Kanal mit einem Einlass und einem Auslass auf. Der Einlass ist fluidleitend mit dem Pumpvolumen verbunden. Der Auslass ist fluidleitend mit der Katheterleitung verbindbar. Der Kanal ist bei der bekannten Pumpvorrichtung in Form eines Schlauchs mit einem vergleichsweise geringen wirksamen Durchmesser ausgebildet und wirkt hierdurch als Strömungswiderstand. Dabei sind die förderdruckerzeugenden und/oder fluidleitenden Komponenten der Pumpvorrichtung zwangsläufig toleranzbehaftet. Dies geht mit einem toleranzbehafteten Förderdruck und damit mit einer toleranzbehafteten Flussrate einher.

Um diese Toleranzen auszugleichen, ist der Kanal der bekannten Pumpvorrichtung derart gestaltet, dass die Förderung des medizinischen Fluids durch den Auslass fertigungsseitig auf die bestimmte Flussrate feinjustierbar ist. Bei der bekannten Pumpvorrichtung wird der Kanal hierzu materialabtragend bearbeitet. Dokument US4176683A offenbart eine Durchflussregelung mir zwei verschiebbaren Körpern. Dokument EP3381489A1 offenbart eine Pumpvorrichtung mit elastischer Membran.

Aufgabe der Erfindung ist es, eine Pumpvorrichtung der eingangs genannten Art zu schaffen, die gegenüber dem Stand der Technik verbesserte Eigenschaften aufweist und insbesondere eine verbesserte fertigungsseitige Feinjustierung der Flussrate ermöglicht.

Diese Aufgabe wird dadurch gelöst, dass die Drosseleinrichtung wenigstens einen ersten Körper und einen zweiten Körper aufweist, wobei der wenigstens eine Kanal zwischen einander gegenüberliegend angeordneten Flächen der beiden Körper ausgebildet ist, und wobei die Flächen zur fertigungsseitigen Feinjustierung der Flussrate derart zueinander relativbeweglich sind, dass eine wirksame Länge des Kanals veränderlich ist. Durch die erfindungsgemäße Lösung kann insbesondere auf eine materialabtragende Bearbeitung des Kanals zur Feinjustierung der Flussrate während der Fertigung verzichtet werden. Bei einer solchen materialabtragenden Bearbeitung fallen Materialpartikel an. Diese können die Pumpvorrichtung verunreinigen, bei deren Gebrauch in das medizinische Fluidleitungssystem gelangen, letztlich ein Gesundheitsrisiko für den Patienten darstellen und müssen daher aufwändig nach der Feinjustierung der Flussrate entfernt werden. Durch die erfindungsgemäße Lösung kann hierauf verzichtet werden. Stattdessen ermöglicht die erfindungsgemäße Lösung eine einfache Änderung der wirksamen Länge - und damit des Strömungswiderstands - des Kanals mittels einer fertigungsseitigen Relativbewegung der einander gegenüberliegend angeordneten Flächen. Die hierdurch bewirkte Änderung der Länge des Kanals ist insbesondere im Gegensatz zu einer materialabtragenden Bearbeitung reversibel. Das heißt nach einem Verkürzen der wirksamen Länge kann die wirksame Länge erforderlichenfalls wieder erhöht werden und umgekehrt. Hierdurch kann bei der Fertigung anfallender Ausschuss vermindert werden. Fertigungsseitige Feinjustierung meint im Sinne der Erfindung, dass zum Zwecke des Toleranzausgleichs in einem gesonderten Verfahrensschritt bei oder nach der Herstellung der Pumpvorrichtung eine individuelle Einstellung der Flussrate auf einen bestimmten Wert erfolgt. In einem späteren gebrauchsfertigen Zustand der Pumpvorrichtung ist die Flussrate demgegenüber vorzugsweise nicht einstellbar. Die einander gegenüberliegend angeordneten Flächen können zur Feinjustierung der Flussrate relativ zueinander linear-, schwenk- und/oder drehbeweglich sein. Entsprechendes gilt für den ersten und den zweiten Körper. Eine erste der Flächen ist dem ersten Körper zugeordnet. Eine zweite der Flächen ist dem zweiten Körper zugeordnet. Der Kanal ist in Umfangsrichtung abschnittsweise von der ersten Fläche und abschnittsweise von der zweiten Fläche berandet. Die wirksame Länge des Kanals erstreckt sich entlang einer Axialrichtung des Kanals zwischen dem Einlass und dem Auslass. Die wirksame Länge kann auch als hydraulisch wirksame Länge bezeichnet werden und ist die Länge des Kanals, entlang derer das medizinische Fluid gefördert wird. Der Kanal kann in beliebiger Form, insbesondere geradlinig, eben oder räumlich gekrümmt, wendelförmig oder spiralförmig, erstreckt sein.

In Ausgestaltung der Erfindung weist der erste Körper eine Zylinderbohrung auf und der zweite Körper ist in Form eines Zylinders ausgebildet, wobei der Zylinder in die Zylinderbohrung eingepasst ist, und wobei der Kanal in Form einer wenigstens eingängigen Wendel in Radialrichtung zwischen der Zylinderbohrung und dem Zylinder ausgebildet ist. Die Wendel kann auch als Helix, Schraube oder zylindrische Spirale bezeichnet werden. Demnach windet sich der Kanal bei dieser Ausgestaltung der Erfindung - vorzugsweise mit konstanter Steigung - um den Zylinder und/oder die Zylinderbohrung. Dies ist insoweit eine besonders vorteilhafte Ausgestaltung der Erfindung, als auf kompaktem Bauraum eine vergleichsweise große wirksame Länge des Kanals erreicht werden kann. Dies erlaubt beispielsweise gegenüber einem geradlinig erstreckten Kanal bei gleicher Drosselwirkung einen vergleichsweise vergrößerten Durchmesser des Kanals. Hierdurch können strömungsdynamische Grenzflächenphänomene, die insbesondere bei Kanälen mit sehr geringem Durchmesser auftreten können, vermieden werden. Solche unerwünschten Grenzflächenphänomene können insbesondere zu einem verzögerten Anlaufen der Förderung des medizinischen Fluids und/oder zu einem vorzeitigen Stopp der Förderung noch vor Erreichen einer gewünschten Fördermenge führen. Durch diese Ausgestaltung der Erfindung kann dem entgegengewirkt werden. Die Zylinderbohrung ist vorzugsweise eine Kreiszylinderbohrung. Der Zylinder ist vorzugsweise ein Kreiszylinder. Die Wendel ist wenigstens eingängig und kann insoweit auch zwei-, drei-, vier- oder darüber hinaus mehrgängig ausgebildet sein. Demgemäß weist die Wendel wenigstens einen ersten Wendelgang auf. Die Wendel kann aber auch zwei, drei, vier oder eine darüber hinausgehende Anzahl von Wendelgängen aufweisen. Zur Feinjustierung der Flussrate sind der Zylinder und die Zylinderbohrung axial und/oder in Umfangsrichtung zueinander relativbeweglich. Vorzugsweise ist der Kanal in Umfangsrichtung abschnittsweise von einer Innenmantelfläche der Zylinderbohrung und abschnittsweise von einer Außenmantelfläche des Zylinders berandet. Bei dieser Ausgestaltung der Erfindung sind die einander gegenüberliegend angeordneten Flächen dementsprechend eine Innenmantelfläche der Zylinderbohrung und eine Außenmantelfläche des Zylinders.

In weiterer Ausgestaltung der Erfindung ist der Kanal in Form einer wenigstens zweigängigen Wendel mit wenigstens einem ersten Wendelgang und einem zweiten Wendelgang ausgebildet. Bei dieser Ausgestaltung der Erfindung kann die Form des Kanals dementsprechend auch als Doppelhelix, zweigängige Schraube oder zweigängige zylindrische Spirale bezeichnet werden. Der erste Wendelgang weist eine erste Windungsrichtung auf. Der zweite Wendelgang weist eine zweite Windungsrichtung auf. Die erste Windungsrichtung und die zweite Windungsrichtung können zueinander entgegengesetzt oder gleichsinnig sein. Der erste Wendelgang weist eine erste Steigung auf. Der zweite Wendelgang weist eine zweite Steigung auf. Die erste Steigung und die zweite Steigung können gleich oder zueinander unterschiedlich bemessen sein. Sofern der erste Wendelgang und der zweite Wendelgang zueinander gleichsinnige Windungsrichtungen und gleiche Steigungen aufweisen, bilden der erste Wendelgang und der zweite Wendelgang keine Überkreuzungen aus und sind insoweit voneinander getrennt. Andernfalls, insbesondere bei zueinander entgegengesetzten Windungsrichtungen, bilden die Wendelgänge Überkreuzungen aus und sind insoweit miteinander verbunden. Diese Ausgestaltung der Erfindung ermöglicht insbesondere eine verbesserte Anpassbarkeit der strömungstechnischen Eigenschaften des Kanals vor dem Hintergrund der zu erzielenden Feinjustierung.

In weiterer Ausgestaltung der Erfindung weisen die Wendelgänge zueinander entgegengesetzte Windungsrichtungen auf. Beispielsweise kann der erste Wendelgang linksgängig sein und der zweite Wendelgang rechtsgängig oder umgekehrt. Dabei bilden die Wendelgänge Überkreuzungen aus. Dies ist besonders vorteilhaft im Hinblick auf eine mögliche Blockade des Kanals. Denn bei einer solchen Blockade des Kanals, die beispielsweise durch einen Fremdkörper oder dergleichen bewirkt sein kann, kann das medizinische Fluid mittels der Überkreuzungen an der Blockade vorbeigeleitet werden. Dies hat auch zur Folge, dass die Drosselwirkung des Kanals durch eine solche etwaige Blockade nur in vergleichsweise geringem Umfang beeinflusst wird.

In weiterer Ausgestaltung der Erfindung ist der Kanal mittels einer wendelförmigen Profilierung ausgebildet, die auf einer Innenmantelfläche der Zylinderbohrung und/oder auf einer Außenmantelfläche des Zylinders angeordnet ist. Die Profilierung kann in Form einer Profilvertiefung und/oder einer Profilerhöhung ausgebildet sein. Beispielsweise kann die Profilierung in Form einer in die Innenmantelfläche und/oder die Außenmantelfläche eingebrachten Profilnut ausgebildet sein. Die Profilierung kann mittels eines im Bereich der Fertigungstechnik grundsätzlich bekannten Verfahrens materialabtragend oder umformend ausgebildet sein. Beispielsweise kann die Profilierung mittels Prägens, Ätzens, Schleifens oder dergleichen ausgebildet sein. Die Profilierung kann eine beliebige Querschnittsform aufweisen. Die Profilierung ist in Umfangsrichtung des Kanals vorzugsweise wenigstens abschnittsweise offen.

In weiterer Ausgestaltung der Erfindung ist die Profilierung mittels Prägens hergestellt. Hierfür geeignete Verfahren sind beispielsweise im Bereich der Schraubenfertigung grundsätzlich bekannt. Durch diese Ausgestaltung der Erfindung können insbesondere enge maßliche Toleranzen bei der Ausbildung des Kanals eingehalten werden. Dies ist im Hinblick auf die Feinjustierung der Flussrate besonders vorteilhaft.

In weiterer Ausgestaltung der Erfindung weist die Profilierung eine Querschnittsform auf, die ausgewählt ist aus einer Gruppe von Querschnittsformen bestehend aus den Formen Rechteck, Dreieck, Kreisabschnitt, Parabel und Trapez. Vorgenannte Querschnittsformen haben sich im Hinblick auf die strömungstechnischen Eigenschaften des Kanals als vorteilhaft erwiesen.

In weiterer Ausgestaltung der Erfindung sind der Zylinder und die Zylinderbohrung derart radial elastisch vorgespannt zusammengefügt, dass eine fluiddichte Pressverbindung erreicht ist. Die fluiddichte Pressverbindung zwischen dem Zylinder und der Zylinderbohrung wirkt einer ungewollten Leckage entgegen und gewährleistet, dass das medizinische Fluid in funktionsgerechter Weise entlang des Kanals förderbar ist. Bei dieser Ausgestaltung hat es sich als vorteilhaft erwiesen, wenn der Zylinder aus einem formstabilen Werkstoff gefertigt ist und die Zylinderbohrung aus einem weichelastischen Werkstoff gefertigt ist oder umgekehrt. Durch eine insoweit ungleiche Paarung der Werkstoffe kann die elastische Vorspannung besonders leicht erreicht werden.

In weiterer Ausgestaltung der Erfindung ist der Zylinder aus einem formstabilen Werkstoff gefertigt und der erste Körper weist einen weichelastischen Schlauchabschnitt auf, der die Zylinderbohrung aufweist. Als formstabiler Werkstoff kann insbesondere Metall, Glas oder ein Kunststoff mit entsprechenden Materialeigenschaften gewählt sein. Der weichelastische Schlauchabschnitt ist vorzugsweise aus einem nachgiebigen Kunststoff gefertigt. Es hat sich gezeigt, dass hierdurch eine besonders vorteilhafte fluiddichte Pressverbindung zwischen dem Zylinder und der Zylinderbohrung erreicht werden kann.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematisch stark vereinfachter Darstellung eine Ausführungsform einer erfindungsgemäßen medizinischen Pumpvorrichtung, die zur Verwendung bei einer Infusionstherapie vorgesehen ist und eine Drosseleinrichtung aufweist,
- Fig. 2: in schematisch stark vereinfachter teilweise geschnittener Detaildarstellung die Drosseleinrichtung der Pumpvorrichtung nach Fig. 1, wobei die Drosseleinrichtung insbesondere einen zweiten Körper in Form eines Zylinders aufweist,
- Fig. 3: in schematisch stark vereinfachter Detaildarstellung eine weitere Ausführungsform eines Zylinders für eine Drosseleinrichtung nach den Fig. 1 und 2,
- Fig. 4: in schematisch stark vereinfachter Detaildarstellung eine weitere Ausführungsform eines Zylinders für eine Drosseleinrichtung nach den Fig. 1 und 2,
- Fig. 5: jeweils in abgeschnittener schematisch stark vereinfachter Querschnittsdarstellung unterschiedliche Querschnittsformen eines Kanals der Drosseleinrichtung nach den Fig. 1 und 2 und
- Fig. 6: in einer Darstellung entsprechend Fig. 2 eine weitere Ausführungsform einer Drosseleinrichtung für die Pumpvorrichtung nach Fig. 1.

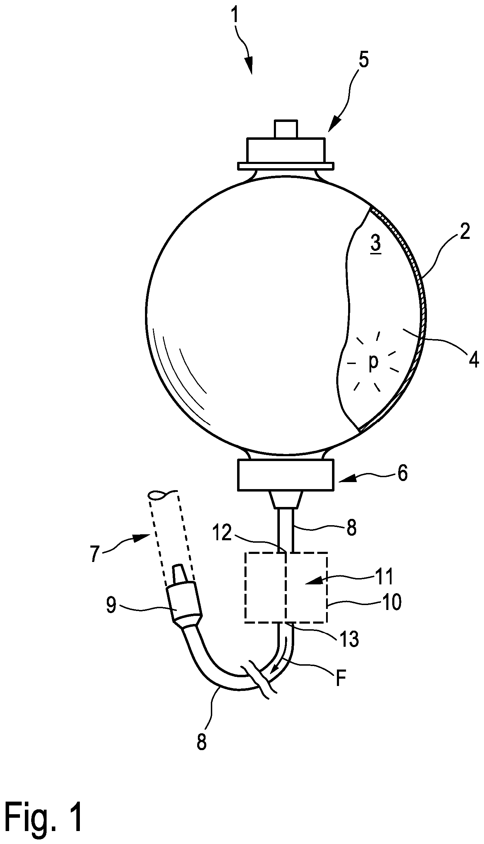

Eine medizinische Pumpvorrichtung 1 nach Fig. 1 ist zur Förderung eines medizinischen Fluids 4 im Rahmen einer ambulanten und/oder stationären Infusionstherapie vorgesehen. Die medizinische Pumpvorrichtung 1 kann auch als elastomere Infusionspumpe oder medizinische Elastomerpumpe bezeichnet werden.

Die medizinische Pumpvorrichtung 1 weist eine elastomere Membran 2 auf, die ein Pumpvolumen 3 zur Aufnahme und Förderung des medizinischen Fluids 4 ausbildet. Das medizinische Fluid ist vorliegend eine nicht näher bezeichnete Medikamentenflüssigkeit. Anhand Fig. 1 ist die medizinische Pumpvorrichtung 1 in einem befüllten Zustand gezeigt. In diesem Zustand ist die elastomere Membran 2 infolge einer mechanischen Einwirkung des medizinischen Fluids 4 ballonartig weichelastisch gedehnt. Dabei ist die elastomere Membran 2 anhand Fig. 1 aus zeichnerischen Gründen mit einer überhöhten Wandungsstärke dargestellt. Demgegenüber ist die Membran 2 in einem nicht mit medizinischem Fluid 4 befüllten Zustand schlaff oder jedenfalls weniger stark elastisch gedehnt. Zum Befüllen des Pumpvolumens 3 bzw. der Membran 2 mit medizinischem Fluid 4 ist ein wiederverschließbarer Einfüllstutzen 5 vorgesehen, der auf grundsätzlich bekannte Weise fluiddicht an die Membran 2 angeschlossen ist.

Die elastisch gedehnte elastomere Membran 2 bewirkt einen Förderdruck p auf das Pumpvolumen 3. Mittels des auf diese Weise bewirkten Förderdrucks p ist das medizinische Fluid 4 über einen auf grundsätzlich bekannte Weise fluiddicht an die elastomere Membran 2 angeschlossenen Durchlassstutzen 6 aus dem Pumpvolumen 3 in ein fluidleitend mit der Pumpvorrichtung 1 verbundenes medizinisches Fluidleitungssystem 7 förderbar. Das medizinische Fluidleitungssystem 7 ist anhand Fig. 1 lediglich stark vereinfacht schematisch und teilweise abgeschnitten strichliert dargestellt. Vorliegend ist das medizinische Fluidleitungssystem eine Patientenleitung 7 eines grundsätzlich bekannten zentralvenösen Katheters, durch den das medizinische Fluid 4 einem zeichnerisch nicht näher dargestellten Patienten verabreicht werden kann. Dabei ist die Pumpvorrichtung 1 vorliegend ausgehend von dem Durchlassstutzen 6 mittels einer Schlauchleitung 8 fluidleitend mit der Patientenleitung 7 verbindbar. Zu diesem Zweck ist die Schlauchleitung 8 an ihrem dem Pumpvolumen 3 zugewandten Stirnende vorliegend unlösbar mit dem Durchlassstutzen 6 verbunden. An ihrem gegenüberliegenden Stirnende weist die Schlauchleitung 8 einen Fluidkonnektor 9 auf. Der Fluidkonnektor ist vorliegend in Form eines grundsätzlich bekannten Luer-Anschlusses 9 ausgebildet. Der Luer-Anschluss 9 ist zur Verbindung mit einem komplementären Luer-Anschluss der Patientenleitung 7 vorgesehen. Bei einer zeichnerisch nicht dargestellten Ausführungsform kann der Fluidkonnektor 9 in Form eines NRFit-Anschlusses ausgebildet sein.

Die medizinische Pumpvorrichtung 1 ist vorliegend derart dimensioniert, dass sie ohne weiteres von einem Patienten am Körper getragen und ohne eine externe Energieversorgung insbesondere im Rahmen einer ambulanten Therapie verwendbar ist. Die medizinische Pumpvorrichtung 1 ist dementsprechend leicht und kompakt dimensioniert, wobei das Pumpvolumen 3 vorliegend nominal mit 400 ml bemessen ist. Es versteht sich, dass das Pumpvolumen 3 auch hiervon abweichend, beispielsweise nominal zwischen 50 ml und 750 ml, bemessen sein kann.

Insbesondere um eine Überdosierung des medizinischen Fluids 4 auszuschließen, ist eine entsprechende Drosselung des Förderdrucks p notwendig. Zu diesem Zweck weist die medizinische Pumpvorrichtung 1 zudem eine Drosseleinrichtung 10 auf. Die Drosseleinrichtung 10 ist vorliegend außerhalb des Pumpvolumens 3 im Bereich der Schlauchleitung 8 angeordnet, was jedoch nicht zwingend der Fall sein muss. Bei einer zeichnerisch nicht näher dargestellten Ausführungsform kann die Drosseleinrichtung 10 beispielsweise in das Pumpvolumen 3 oder den Durchlassstutzen 6 integriert sein. Die Drosseleinrichtung 10 weist einen anhand Fig. 1 schematisch stark vereinfacht dargestellten Kanal 11 auf. Der Kanal weist einen Einlass 12 und einen Auslass 13 auf. Der Einlass 12 ist vorliegend über die Schlauchleitung 8 und den Durchlassstutzen 6 fluidleitend mit dem Pumpvolumen 3 verbunden. Der Auslass 13 ist vorliegend über die Schlauchleitung 8 und den Fluidkonnektor 9 fluidleitend mit der Patientenleitung 7 verbunden.

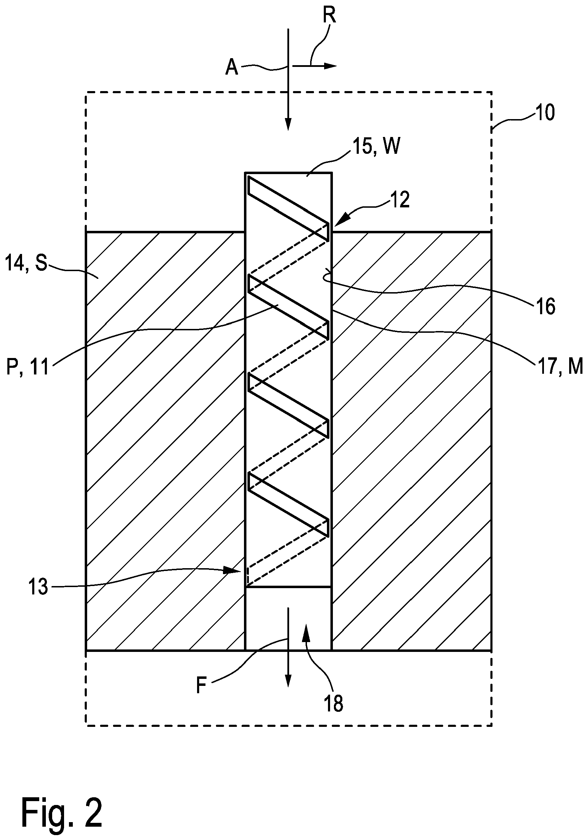

Insbesondere die elastomere Membran 2 unterliegt herstellungsbedingten maßlichen Toleranzen. Auch die Eigenschaften des Werkstoffs der elastomeren Membran 2 können gewissen Toleranzen unterliegen. Diese Toleranzen bewirken einen toleranzbehafteten Förderdruck p und damit eine mit Toleranzen behaftete Förderung des medizinischen Fluids 4. Dies ist aus medizinischer Sicht unerwünscht, da eine mit der medizinischen Pumpvorrichtung 1 verabreichbare Flussrate F des medizinischen Fluids 4 möglichst exakt erreicht werden muss. Um dies zu gewährleisten, ist der Kanal 11 derart gestaltet, dass die Förderung des medizinischen Fluids 4 durch den Auslass 13 fertigungsseitig auf eine bestimmte Flussrate F feinjustierbar ist. Durch die fertigungsseitige Feinjustage mittels des Kanals 11 können demnach insbesondere maßliche oder werkstoffseitige Toleranzen der elastomeren Membran 2 ausgeglichen werden. Weitere konstruktive und funktionale Merkmale der Drosseleinrichtung 10 sind insbesondere anhand Fig. 2 ersichtlich.

Die Drosseleinrichtung 10 weist einen ersten Körper 14 und einen zweiten Körper 15 auf. Der Kanal 11 ist zwischen einander gegenüberliegend angeordneten Flächen 16, 17 der beiden Körper 14, 15 ausgebildet. Eine erste Fläche 16 ist dem ersten Körper 14 zugeordnet. Eine zweite Fläche 17 ist dem zweiten Körper 15 zugeordnet. Zur Feinjustierung der Flussrate F und damit zum vorbeschriebenen Toleranzausgleich sind die beiden Flächen 16, 17 derart zueinander relativbeweglich, dass eine wirksame Länge des Kanals 11 veränderlich ist. Die nicht näher bezeichnete wirksame Länge des Kanals 11 erstreckt sich zwischen dem Einlass 12 und dem Auslass 13. Je geringer die wirksame Länge des Kanals 11, desto geringer ist ein Strömungswiderstand und damit eine Drosselwirkung auf die Förderung des medizinischen Fluids 4. Je größer die wirksame Länge des Kanals 11 ist, desto größer ist der Strömungswiderstand und damit die Drosselwirkung auf die Förderung des medizinischen Fluids 4. Demgemäß kann mit einer Verringerung der wirksamen Länge eine Erhöhung der Flussrate F und in umgekehrter Weise mit einer Vergrößerung der wirksamen Länge eine Verringerung der Flussrate F zum Zwecke der Feinjustierung bewirkt werden. Zur Feinjustierung der Flussrate F wird der zweite Körper 15 vorliegend entlang einer Axialrichtung A relativ zu dem ersten Körper 14 bewegt. Wie anhand Fig. 2 ersichtlich ist, ändert sich hierdurch die wirksame Länge des zwischen den Flächen 16, 17 ausgebildeten Kanals 11 und damit dessen Strömungswiderstand. Wird der zweite Körper 15 ausgehend von der anhand Fig. 2 ersichtlichen Konfiguration in Axialrichtung A weiter - in Bezug auf die Zeichenebene der Fig. 2 - nach unten bewegt, bewirkt dies eine Vergrößerung der wirksamen Länge des Kanals 11. Wird der zweite Körper 15 hierzu entgegengesetzt nach oben bewegt, bewirkt dies eine Verringerung der wirksamen Länge des Kanals 11. Vorliegend ist vorgesehen, dass die vorbeschriebene Anpassung der wirksamen Länge, d. h. die Feinjustierung, fertigungsseitig und damit in einem gesonderten Herstellungs- und/oder Montageschritt erfolgt. Eine Anpassung der wirksamen Länge des Kanals 11 in einem gebrauchsfertigen Zustand der Pumpvorrichtung 1 ist demgegenüber vorliegend nicht vorgesehen.

Vorliegend weist der erste Körper 14 eine Zylinderbohrung 18 auf und der zweite Körper ist in Form eines Zylinders 15 ausgebildet und in die Zylinderbohrung 18 eingepasst. Dabei ist der Kanal in Form einer wenigstens eingängigen Wendel 11 in Radialrichtung R zwischen der Zylinderbohrung 18 und dem Zylinder 15 ausgebildet. Die Wendel 11 kann auch als Helix, Schraube oder zylindrische Spirale bezeichnet werden. Durch diese Ausgestaltung des Kanals 11 kann auf einem vergleichsweise kompakten Bauvolumen eine große wirksame Länge des Kanals 11 erreicht werden. Im Vergleich zu einem beispielsweise in Axialrichtung A geradlinig zwischen dem Einlass 12 und dem Auslass 13 erstreckten Kanal kann bei gleichem Strömungswiderstand und damit bei gleicher Drosselwirkung ein vergleichsweise größerer wirksamer Querschnitt realisiert werden. Hierdurch kann Grenzflächenphänomenen entgegengewirkt werden, die mit einem sehr geringen wirksamen Durchmesser einhergehen können. Insbesondere kann durch die Gestaltung des Kanals in Form der Wendel 11 ein verzögertes Anlaufen der Förderung und/oder ein vorzeitiger Stopp der Förderung infolge besagter Grenzflächenphänomene vermieden werden.

Die Zylinderbohrung 18 ist vorliegend kreiszylindrisch ausgebildet. Dementsprechend ist der Zylinder 15 ein Kreiszylinder. Die Wendel 11 erstreckt sich in Umfangsrichtung umlaufend gewendelt mit einer nicht näher bezeichneten Steigung gegenüber der Axialrichtung A über die gesamte Länge des Zylinders 15. Die Wendel 11 weist vorliegend eine konstante Steigung auf.

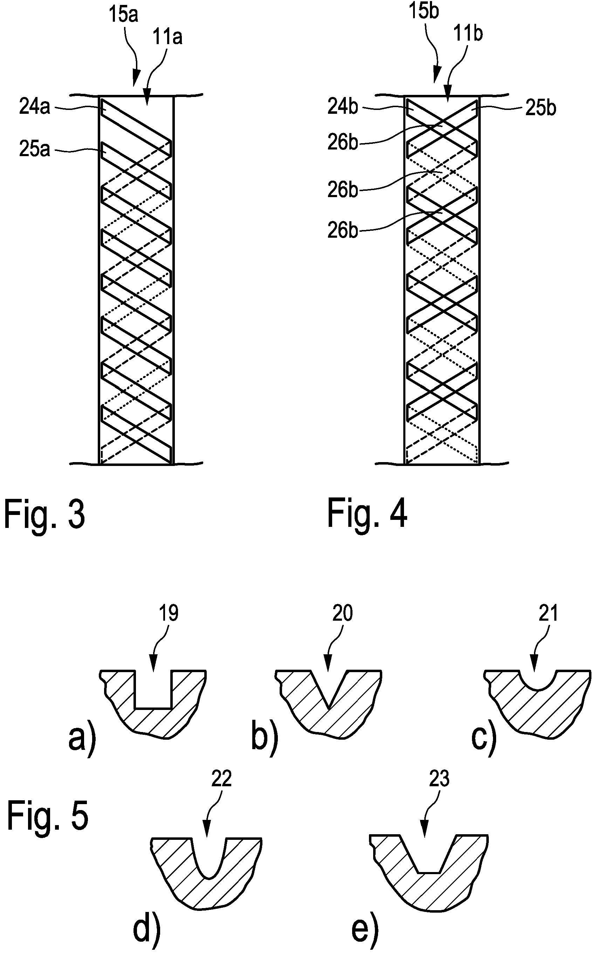

Dabei ist der Kanal 11 mittels einer wendelförmigen Profilierung P ausgebildet, die vorliegend auf einer Außenmantelfläche M des Zylinders 15 angeordnet ist. Die Profilierung P weist eine rechteckige Querschnittsform auf (Fig. 5a) und ist mittels Prägens hergestellt. Hierzu ist der Zylinder 15 aus einem formstabilen Werkstoff W hergestellt, der mittels eines im Bereich der Fertigungstechnik grundsätzlich bekannten Verfahrens prägbar ist.

Abweichend von der anhand Fig. 5a) ersichtlichen Querschnittsform in Form eines Rechtecks 19 sind alternativ weitere Querschnittsformen möglich und anhand der weiteren Fig. 5b) bis 5e) zeichnerisch dargestellt. So kann die Profilierung P alternativ eine Querschnittsform in Form eines Dreiecks 20, eines Kreisabschnitts 21, einer Parabel 22 oder eines Trapezes 23 aufweisen.

Der erste Körper 14 weist einen weichelastischen Schlauchabschnitt S auf, durch den die Zylinderbohrung 18 erstreckt ist. Dadurch ist die Zylinderbohrung 18 wenigstens in Radialrichtung R weichelastisch nachgiebig ausgebildet. Der Zylinder 15 ist unter radial elastischer Vorspannung der Zylinderbohrung 18 in dieselbe eingepresst, so dass eine fluiddichte Pressverbindung zwischen dem Zylinder 15 und der Zylinderbohrung 18 erreicht ist. Dies gewährleistet, dass das medizinische Fluid 4 in funktionsgerechter Weise entlang des Kanals 11 gefördert und nicht etwa am Außenumfang des Zylinders 15 vorbeigepresst wird.

Anhand Fig. 3 ist eine weitere Ausführungsform eines Zylinders 15a ersichtlich, der anstelle des Zylinders 15 in die Zylinderbohrung 18 einpassbar ist. Der Zylinder 15a entspricht im Hinblick auf seine konstruktiven und funktionellen Merkmale im Wesentlichen dem Zylinder 15. Zur Vermeidung von Wiederholungen wird auf die Offenbarung im Zusammenhang mit dem Zylinder 15 verwiesen, die in entsprechender Weise für den Zylinder 15a gilt. Nachfolgend wird lediglich auf die Unterschiede des Zylinders 15a zu dem Zylinder 15 eingegangen. Im Unterschied zu dem Zylinder 15 ist ein Kanal 11a vorgesehen. Der Kanal ist in Form einer zweigängigen Wendel 11a ausgebildet und weist einen ersten Wendelgang 24a und einen zweiten Wendelgang 25a auf. Der erste Wendelgang 24a weist eine erste nicht näher bezeichnete Windungsrichtung auf. Der zweite Wendelgang 25a weist eine zweite nicht näher bezeichnete Windungsrichtung auf. Dabei stimmen die beiden Windungsrichtungen überein. Zusätzlich weisen die Wendelgänge 24a, 25a eine gleiche Steigung auf. Durch die zweigängige Gestaltung der Wendel 11a kann insbesondere einer unerwünschten Blockade des Kanals 11a entgegengewirkt werden. Ist beispielsweise der Wendelgang 24a durch einen Fremdkörper verstopft, kann das medizinische Fluid dennoch entlang des weiteren Wendelgangs 25a gefördert werden und umgekehrt.

Anhand Fig. 4 ist eine weitere Ausführungsform eines Zylinders 15b ersichtlich. Zur Vermeidung von Wiederholungen wird wiederum auf die Offenbarung im Zusammenhang mit dem Zylinder 15 und dem Zylinder 15a verwiesen, die jeweils in entsprechender Weise für den Zylinder 15b gilt. Nachfolgend wird lediglich auf die Unterschiede des Zylinders 15b zu dem Zylinder 15a eingegangen. Der Zylinder 15b weist wiederum einen Kanal in Form einer zweigängigen Wendel 11b auf, die mit einem ersten Wendelgang 24b und einem zweiten Wendelgang 25b ausgebildet ist. Die Wendelgänge 24b, 25b weisen zueinander entgegengesetzte nicht näher bezeichnete Windungsrichtungen auf. Hierdurch bilden die Wendelgänge 24b, 25b Überkreuzungen 26b aus. Die Wendelgänge 24b sind somit fluidleitend miteinander verbunden.

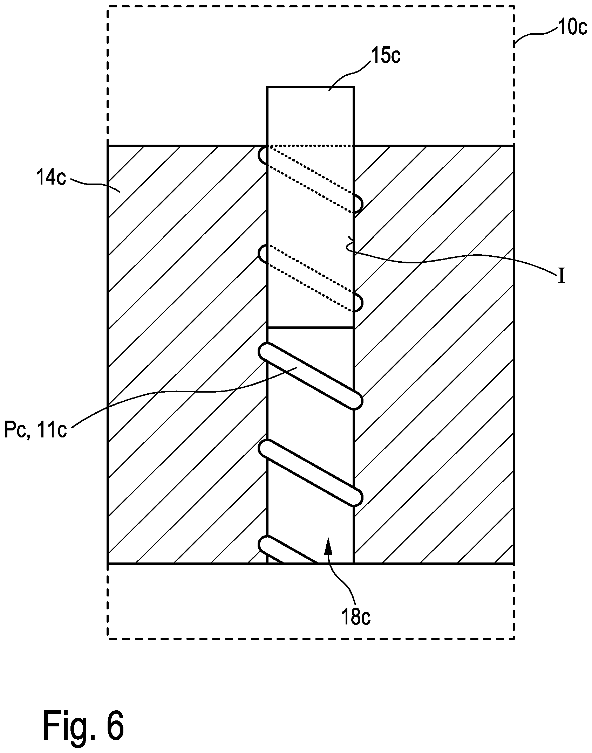

Anhand Fig. 6 ist eine Drosseleinrichtung 10c schematisch stark vereinfacht dargestellt. Die Drosseleinrichtung 10c kann anstelle der Drosseleinrichtung 10 für die medizinische Pumpvorrichtung 1 nach Fig. 1 verwendet werden. Dabei weist die Drosseleinrichtung 10c im Hinblick auf ihre konstruktive Gestaltung und Funktion eine überwiegende Übereinstimmung mit der Drosseleinrichtung 10 auf. Zur Vermeidung von Wiederholungen wird deshalb auf die Offenbarung im Zusammenhang mit der Drosseleinrichtung 10 verwiesen, die in entsprechender Weise für die Drosseleinrichtung 10c gilt. Die Drosseleinrichtung 10c weist einen Kanal 11c auf. Der Kanal ist wiederum in Form einer eingängigen Wendel 11c zwischen einer Zylinderbohrung 18c und einem Zylinder 15c ausgebildet. Der Zylinder 15c ist aus zeichnerischen Gründen und zur besseren Erkennbarkeit der Wendel 11c in axialer Richtung verkürzt dargestellt. Im Unterschied zu dem Kanal 11 ist der Kanal 11c mittels einer Profilierung Pc ausgebildet, die auf einer Innenmantelfläche I der Zylinderbohrung 18c angeordnet ist. Dementsprechend ist die Profilierung Pc in die Innenmantelfläche I eingeprägt. Die Profilierung Pc weist vorliegend eine Querschnittsform in Form eines Kreisabschnitts 21 gemäß Fig. 5c) auf, was jedoch nicht zwingend der Fall sein muss. Anstelle des Kreisabschnitts 21 können auch die weiteren Querschnittsformen gemäß Fig. 5 oder eine anderweitige Querschnittsform vorgesehen sein.

## Patentansprüche

1. Medizinische Pumpvorrichtung (1) zur Förderung eines medizinischen Fluids (4), aufweisend
- eine elastomere Membran (2), die ein Pumpvolumen (3) zur Aufnahme und Förderung des medizinischen Fluids (4) ausbildet,
- wobei die elastomere Membran (2) in einem wenigstens teilweise mit dem medizinischen Fluid (4) befüllten Füllzustand des Pumpvolumens (3) elastisch gedehnt ist,
- und wobei mittels der elastisch gedehnten Membran (2) ein Förderdruck (p) auf das Pumpvolumen (3) zur Förderung des medizinischen Fluids (4) in ein fluidleitend mit dem Pumpvolumen (3) verbindbares medizinisches Fluidleitungssystem (7) bewirkt ist,
- und aufweisend eine Drosseleinrichtung (10, 10c) mit wenigstens einem Kanal (11, 11a bis 11c),
- wobei der Kanal (11, 11a bis 11c) einen Einlass (12), der fluidleitend mit dem Pumpvolumen (3) verbunden ist, und einen Auslass (13), der fluidleitend mit dem medizinischen Fluidleitungssystem (7) verbindbar ist, aufweist,
- und wobei der wenigstens eine Kanal (11, 11a bis 11c) derart gestaltet ist, dass die Förderung des medizinischen Fluids (4) durch den Auslass (13) fertigungsseitig auf eine bestimmte Flussrate (F) feinjustierbar ist,
- **dadurch gekennzeichnet, dass** die Drosseleinrichtung (10, 10c) wenigstens einen ersten Körper (14, 14c) und einen zweiten Körper (15, 15a bis 15c) aufweist, wobei der wenigstens eine Kanal (11, 11a bis 11c) zwischen einander gegenüberliegend angeordneten Flächen (16, 17) der beiden Körper (14, 14c, 15, 15a bis 15c) ausgebildet ist, und wobei die Flächen (16, 17) zur fertigungsseitigen Feinjustierung der Flussrate (F) derart zueinander relativbeweglich sind, dass eine wirksame Länge des Kanals (11, 11a bis 11c) veränderlich ist, und dass der erste Körper (14, 14c) eine Zylinderbohrung (18, 18c) aufweist und der zweite Körper in Form eines Zylinders (15, 15a bis 15c) ausgebildet ist, wobei der Zylinder (15, 15a bis 15c) in die Zylinderbohrung (18, 18c) eingepasst ist, und wobei der Kanal in Form einer wenigstens eingängigen Wendel (11, 11a bis 11c) in Radialrichtung (R) zwischen der Zylinderbohrung (18, 18c) und dem Zylinder (15, 15a bis 15c) ausgebildet ist.

2. Medizinische Pumpvorrichtung (1) nach Anspruch 1, **dadurch gekennzeichnet, dass** der Kanal in Form einer wenigstens zweigängigen Wendel (11a, 11b) mit wenigstens einem ersten Wendelgang (24a, 24b) und einem zweiten Wendelgang (25a, 25b) ausgebildet ist.

3. Medizinische Pumpvorrichtung (1) nach Anspruch 2, **dadurch gekennzeichnet, dass** die Wendelgänge (24b, 25b) zueinander entgegengesetzte Windungsrichtungen aufweisen.

4. Medizinische Pumpvorrichtung (1) nach einem der vorhergehenden Ansprüche, **dadurch gekennzeichnet, dass** der Kanal (11, 11a bis 11c) mittels einer wendelförmigen Profilierung (P, Pc) ausgebildet ist, die auf einer Innenmantelfläche (I) der Zylinderbohrung (18c) und/oder auf einer Außenmantelfläche (M) des Zylinders (15, 15a, 15b) angeordnet ist.

5. Medizinische Pumpvorrichtung (1) nach Anspruch 4, **dadurch gekennzeichnet, dass** die Profilierung (P, Pc) mittels Prägens hergestellt ist.

6. Medizinische Pumpvorrichtung (1) nach Anspruch 4 oder 5, **dadurch gekennzeichnet, dass** die Profilierung (P, Pc) eine Querschnittsform aufweist, die ausgewählt ist aus einer Gruppe von Querschnittsformen bestehend aus den Formen Rechteck (19), Dreieck (20), Kreisabschnitt (21), Parabel (22) und Trapez (23).

7. Medizinische Pumpvorrichtung (1) nach einem der vorhergehenden Ansprüche, **dadurch gekennzeichnet, dass** der Zylinder (15, 15a bis 15c) und die Zylinderbohrung (18, 18c) derart radial elastisch vorgespannt zusammengefügt sind, dass eine fluiddichte Pressverbindung erreicht ist.

8. Medizinische Pumpvorrichtung (1) nach Anspruch 7, **dadurch gekennzeichnet, dass** der Zylinder (15, 15a, 15b) aus einem formstabilen Werkstoff (W) gefertigt ist und dass der erste Körper (14) einen weichelastischen Schlauchabschnitt (S) aufweist, der die Zylinderbohrung (18) aufweist.

## Claims

1. Medical pump device (1) for delivering a medical fluid (4), having
- an elastomeric membrane (2), which forms a pump volume (3) for receiving and delivering the medical fluid (4),
- wherein the elastomeric membrane (2) is elastically expanded in a filling state of the pump volume (3) at least partially filled with the medical fluid (4),
- and wherein, by means of the elastically expanded membrane (2), a delivery pressure (p) is exerted on the pump volume (3) in order to deliver the medical fluid (4) into a medical fluid-line system (7) connectable in a fluid-conducting manner to the pump volume (3),
- and having a throttle device (10, 10c) with at least one channel (11, 11a to 11c),
- wherein the channel (11, 11a to 11c) has an inlet (12), which is connected in a fluid-conducting manner to the pump volume (3), and an outlet (13), which is connectable in a fluid-conducting manner to the medical fluid-line system (7),
- and wherein the at least one channel (11, 11a to 11c) is designed in such a way that the delivery of the medical fluid (4) through the outlet (13) can be finely adjusted to a defined flow rate (F) at the time of manufacture,
- **characterized in that** the throttle device (10, 10c) has at least a first body (14, 14c) and a second body (15, 15a to 15c), wherein the at least one channel (11, 11a to 11c) is formed between oppositely arranged surfaces (16, 17) of the two bodies (14, 14c, 15, 15a to 15c), and wherein the surfaces (16, 17) are movable relative to each other for the fine adjustment of the flow rate (F) at the time of manufacture, in such a way that an effective length of the channel (11, 11a to 11c) is variable, and **in that** the first body (14, 14c) has a cylinder bore (18, 18c), and the second body is designed in the form of a cylinder (15, 15a to 15c), wherein the cylinder (15, 15a to 15c) is fitted into the cylinder bore (18, 18c), and wherein the channel is designed in the form of an at least single-flight helix (11, 11a to 11c) in a radial direction (R) between the cylinder bore (18, 18c) and the cylinder (15, 15a to 15c).

2. Medical pump device (1) according to Claim 1, **characterized in that** the channel is designed in the form of an at least double-flight helix (11a, 11b) with at least a first helical flight (24a, 24b) and a second helical flight (25a, 25b).

3. Medical pump device (1) according to Claim 2, **characterized in that** the helical flights (24b, 25b) have mutually opposite winding directions.

4. Medical pump device (1) according to any one of the preceding claims, **characterized in that** the channel (11, 11a to 11c) is designed by means of a helical profiling (P, Pc), which is arranged on an inner lateral face (I) of the cylinder bore (18c) and/or on an outer lateral face (M) of the cylinder (15, 15a, 15b).

5. Medical pump device (1) according to Claim 4, **characterized in that** the profiling (P, Pc) is produced by means of embossing.

6. Medical pump device (1) according to Claim 4 or 5, **characterized in that** the profiling (P, Pc) has a cross-sectional shape that is chosen from a group of cross-sectional shapes consisting of a rectangle (19), triangle (20), circle segment (21), parabola (22) and trapezoid (23).

7. Medical pump device (1) according to any one of the preceding claims, **characterized in that** the cylinder (15, 15a to 15c) and the cylinder bore (18, 18c) are joined together with radial elastic pretensioning in such a way that a fluid-tight interference fit is obtained.

8. Medical pump device (1) according to Claim 7, **characterized in that** the cylinder (15, 15a, 15b) is made from a dimensionally stable material (W), and **in that** the first body (14) has a flexible hose portion (S) that has the cylinder bore (18).

## Revendications

1. Dispositif de pompage médical (1) pour le transport d'un fluide médical (4), présentant
- une membrane élastomère (2) réalisant un volume de pompage (3) pour recevoir et transporter le fluide médical (4),
- la membrane élastomère (2) étant étirée élastiquement dans un état de remplissage du volume de pompage (3) au moins partiellement rempli avec le fluide médical (4),
- et, au moyen de la membrane (2) étirée élastiquement, une pression de transport (p) étant exercée sur le volume de pompage (3) pour transporter le fluide médical (4) dans un système de conduite de fluide médical (7) pouvant être relié fluidiquement au volume de pompage (3),
- et présentant un appareil d'étranglement (10, 10c) avec au moins un canal (11, 11a à 11c),
- le canal (11, 11a à 11c) présentant une entrée (12) qui est reliée fluidiquement au volume de pompage (3) et une sortie (13) qui peut être reliée fluidiquement au système de conduite de fluide médical (7),
- et l'au moins un canal (11, 11a à 11c) étant conçu de telle sorte que le transport du fluide médical (4) à travers la sortie (13) peut être ajusté finement côté fabrication à un débit déterminé (F),
- **caractérisé en ce que** l'appareil d'étranglement (10, 10c) présente au moins un premier corps (14, 14c) et un deuxième corps (15, 15a à 15c), l'au moins un canal (11, 11a à 11c) étant réalisé entre des surfaces (16, 17) des deux corps (14, 14c, 15, 15a à 15c) agencées en face l'une de l'autre, et les surfaces (16, 17) étant mobiles l'une par rapport à l'autre pour l'ajustement fin du débit (F) côté fabrication, de telle sorte qu'une longueur efficace du canal (11, 11a à 11c) est variable, et **en ce que** le premier corps (14, 14c) présente un alésage cylindrique (18, 18c) et le deuxième corps est réalisé sous la forme d'un cylindre (15, 15a à 15c), le cylindre (15, 15a à 15c) étant emboîté dans l'alésage cylindrique (18, 18c), et le canal étant réalisé sous la forme d'une hélice à au moins une spire (11, 11a à 11c) dans la direction radiale (R) entre l'alésage cylindrique (18, 18c) et le cylindre (15, 15a à 15c).

2. Dispositif de pompage médical (1) selon la revendication 1, **caractérisé en ce que** le canal est réalisé sous la forme d'une hélice (11a, 11b) à au moins deux spires, avec au moins une première spire (24a, 24b) et une deuxième spire (25a, 25b).

3. Dispositif de pompage médical (1) selon la revendication 2, **caractérisé en ce que** les spires (24b, 25b) ont des directions d'enroulement opposées l'une à l'autre.

4. Dispositif de pompage médical (1) selon l'une quelconque des revendications précédentes, **caractérisé en ce que** le canal (11, 11a à 11c) est réalisé au moyen d'un profilage hélicoïdal (P, Pc) agencé sur une surface d'enveloppe intérieure (I) de l'alésage cylindrique (18c) et/ou sur une surface d'enveloppe extérieure (M) du cylindre (15, 15a, 15b).

5. Dispositif de pompage médical (1) selon la revendication 4, **caractérisé en ce que** le profilage (P, Pc) est fabriqué par estampage.

6. Dispositif de pompage médical (1) selon la revendication 4 ou 5, **caractérisé en ce que** le profilage (P, Pc) présente une forme de section transversale choisie dans un groupe de formes de section transversale constitué par les formes rectangle (19), triangle (20), segment de cercle (21), parabole (22) et trapèze (23).

7. Dispositif de pompage médical (1) selon l'une quelconque des revendications précédentes, **caractérisé en ce que** le cylindre (15, 15a à 15c) et l'alésage cylindrique (18, 18c) sont assemblés avec une précontrainte élastique radiale de telle sorte qu'on obtient une liaison par pression étanche aux fluides.

8. Dispositif de pompage médical (1) selon la revendication 7, **caractérisé en ce que** le cylindre (15, 15a, 15b) est fabriqué dans un matériau indéformable (W) et **en ce que** le premier corps (14) présente une section de tuyau souple et élastique (S) qui présente l'alésage cylindrique (18).
